# EUROPEAN PATENT APPLICATION

(11) **EP 4 692 357 A1**
(43) Date of publication of application: **11.02.2026**
(21) Application number: 24778044.8
(22) Date of filing: 27.03.2024
(51) Int. Cl.: C12N 15/70, C12R 1/19, C12N 9/88, C12N 15/113, C12P 13/06, C12P 13/08, C12P 13/10, C12P 13/22, C12N 15/60, C12N 1/21

(54) **ISOCITRATE LYASE AND USE THEREOF IN PREPARATION OF L-AMINO ACID**

(30) Priority: 29.03.2023 CN 202310316337
(71) Applicant: Ningxia Eppen Biotech Co. Ltd, Ningxia 750100 (CN)
(72) Inventor: WANG, Jiwei, Yinchuan, Ningxia 750100 (CN); WEI, Aiying, Yinchuan, Ningxia 750100 (CN); ZHAO, Chunguang, Yinchuan, Ningxia 750100 (CN); SU, Houbo, Yinchuan, Ningxia 750100 (CN); ZHANG, Ying, Yinchuan, Ningxia 750100 (CN); TIAN, Bin, Yinchuan, Ningxia 750100 (CN); ZHANG, Xiaoqin, Yinchuan, Ningxia 750100 (CN); MENG, Gang, Yinchuan, Ningxia 750100 (CN); MA, Congling, Yinchuan, Ningxia 750100 (CN)
(74) Representative: Winter, Brandl - Partnerschaft mbB
(86) International application number: PCT/CN2024/083987
(87) International publication number: WO 2024/199258

(57) **Abstract**

Provided are isocitrate lyase and a use thereof in preparation of L-amino acid. Specifically, provided is a use of a protein having an amino acid sequence of SEQ ID No. 2 or a substance for regulating the activity and/or content of the protein in constructing a genetically engineered bacterium for producing L-amino acid. An aceA gene mutant engineered strain obtained by introducing a point mutation (C-T) to position 1111 of an aceA gene coding region of an L-amino acid high-yield strain and wild-type Escherichia coli W3110, and an engineered strain obtained by knocking out an aceA gene are constructed. Experiments show that the aceA gene and a variant thereof such as an aceAQ371* gene participate in biosynthesis of L-amino acid, and knocking out or weakening the aceA gene facilitates accumulation of L-amino acid. The aceA gene and the variant thereof can be used to construct a genetically engineered strain for producing L-amino acid so as to promote the increase of the yield of L-amino acid.

## Description

### CROSS-REFERENCE TO RELATED APPLICATION

This application claims priority to Chinese Patent Application No. 202310316337.2, filed on March 29, 2023 and entitled "EFFECTS OF ISOCITRATE LYASE GENE ACEA ON SYNTHESIS OF L-AMINO ACID", which is herein incorporated by reference in its entirety.

### Technical Field

The present application belongs to the field of biotechnology and relates to an isocitrate lyase and a use thereof in the preparation of L-amino acid, and more particularly, to the effects of an isocitrate lyase gene aceA on the synthesis of amino acids such as L-alanine, L-valine, L-arginine, L-tryptophan, and L-threonine.

### Background Art

L-amino acid has been used in the animal feed, as well as pharmaceutical and cosmetic industries. A microbial fermentation method is currently the most widely used method for producing L-amino acid. The fermentation production performance of amino acid-producing bacteria is a key factor that determines whether the fermentation method can be industrially applied on a large scale. At present, there are still a few amino acid varieties that have not yet been produced through a fermentation method due to the lack of production strains with excellent fermentation performances. For amino acid-producing strains that have been produced through the fermentation method, in order to reduce the production cost, the acid production level and the sugar-to-acid conversion rate still need to be further improved.

High-quality producing strains are the guarantee for improving the yield and quality of amino acids. With the development of recombinant DNA technologies and the acquisition of relevant microbial genome information, a genetic engineering breeding technology based on a metabolic engineering principle has gradually become the mainstream. The metabolic pathways and metabolic networks of microorganisms can be purposefully modified to artificially change the metabolic regulatory mechanisms of the microorganisms, such that a metabolic flow within the microorganisms flows toward a required direction. Excessive accumulation of amino acids, significant increase in the yield of amino acids, and cost reduction are all of great significance to accelerate the process of L-amino acid industrialization.

The isocitrate lyase aceA is a key enzyme in the glyoxylate cycle of oilseed crop seeds. Currently, there is no research on the functions of aceA in the production of L-amino acid.

### Summary of the Invention

The technical problem to be solved by the present application is how to increase the yield of L-amino acid in microorganisms through genetic modification of genes. The technical problem to be solved is not limited to the described technical subject. Other technical themes which are not mentioned herein can be clearly understood by those skilled in the art through the following description.

To solve the above technical problems, the present application first provides a use of a protein or a substance that regulates the activity and/or content of the protein. The use may include any of the followings:
A1) a use in the construction of genetically engineered bacteria that produces L-amino acid;
A2) a use in the preparation of L-amino acid;
A3) a use in the regulation of the yield of L-amino acid in a microorganism;
the protein is named as an aceA protein and may include any of the followings:
B1) a protein comprising an amino acid sequence as shown in SEQ ID No. 2;
B2) a protein which is obtained by substitution and/or deletion and/or addition of an amino acid residue in the amino acid sequence as shown in SEQ ID No, 2, has the identity of 90% or more with the protein shown in B1) and has the same function as the protein shown in B1); and
B3) a fusion protein which is obtained by linking a tag to an N-terminal and/or a C-terminal of B1) or B2) and has the same function as that of B1) or B2).

The linking in B3) is implemented through a peptide bond.

The tag includes, but is not limited to: a glutathione S-transferase (GST)-tag protein, a His-tag protein, a maltose-binding protein (MBP)-tag protein, a Flag-tag protein, a SUMO-tag protein, an HA-tag protein, an Myc-tag protein, an enhanced green fluorescent protein (eGFP), an enhanced cyan fluorescent protein (eCFP), an enhanced yellow-green fluorescent protein (eYFP), an mCherry (monomeric red fluorescent protein), or an AviTag-tag protein.

A person of ordinary skill in the art can readily mutate a nucleotide sequence that codes the protein aceA in the present application by using known methods, such as directed evolution or point mutation. Those artificially modified nucleotides that have the identity of 75% or more with the nucleotide sequence of the protein aceA isolated from the present application are all derived from the nucleotide sequences of the present application and are equivalent to the sequences of the present application, as long as they code the protein aceA and have the function of the protein aceA.

The above identity of 75% or more may be the identity of 80%, 85%, 90% or 95% or more.

As used herein, the identity refers to the identity of an amino acid sequence or nucleotide sequence. The identity of the amino acid sequence may be determined using a homology search site on Internet, such as a BLAST page of the NCBI homepage website. For example, in advanced BLAST2.1, with blastp as a program, an Expect value is set as 10, and all Filters are set as OFF; with BLOSUM62 as Matrix, the Gap existence cost, the Per residue gap cost, and the Lambda ratio are set as **11,** 1 and 0.85 (default values), respectively; and the identity of the amino acid sequence is searched and calculated, and then the value of identity (%) can be obtained.

As used herein, the above identity of 80% or more may be the identity of at least 80%, 81%, 82%, 83%, 84%, 85%, 86%, 87%, 88%, 89%, 90%, 91%, 92%, 93%, 94%, 95%, 96%, 97%, 98% or 99%. The identity of 85% or more may be the identity of at least 85%, 86%, 87%, 88%, 89%, 90%, 91%, 92%, 93%, 94%, 95%, 96%, 97%, 98% or 99%. The identity of 90% or more may be the identity of at least 90%, 91%, 92%, 93%, 94%, 95%, 96%, 97%, 98% or 99%. The identity of 95% or more may be the identity of at least 95%, 96%, 97%, 98% or 99%.

As used herein, the substance that regulates the activity and/or content of the protein may be a substance that regulates the expression of a gene, wherein the gene codes the protein aceA.

As described above, the substance that regulates the gene expression may be a substance that regulates at least one of the following six types of regulation: 1) regulation at a gene transcription level; 2) regulation performed after the gene transcription (that is, regulation of modification, splicing and/or processing of a transcription product of the gene); 3) regulation of RNA transport of the gene (that is, regulation of mRNA transport of the gene from the nucleus to the cytoplasm); 4) regulation of translation of the gene; 5) regulation of mRNA degradation of the gene; and 6) regulation after the translation of the gene (including regulation of the activity of the protein translated by the gene, such as regulation of protein precursor processing, protein transport, protein degradation, and/or protein folding).

The substance that regulates gene expression may be specified as any of the biomaterials described herein.

Further, the substance that regulates gene expression may be a substance (including a nucleic acid molecule or vector) that inhibits or reduces or down-regulates the expression of a coding gene of the protein aceA. The substance that inhibits or reduces or down-regulates the expression of the coding gene of the protein aceA may be a reagent from which the gene (aceA gene) is knocked out, such as a reagent from which the gene is knocked out by CRISPR-Cas9, or a reagent from which the gene is knocked out by homologous recombination. The reagent that inhibits or reduces the gene expression may contain a polynucleotide that targets the gene, such as siRNA, shRNA, sgRNA, miRNA, or antisense RNA.

The present application further provides a use of a nucleic acid molecule that codes the protein aceA. The use includes any of the followings:
D1) a use in the construction of genetically engineered bacteria that produces L-amino acid;
D2) a use in the preparation of L-amino acid; and
D3) a use in the regulation of the yield of L-amino acid in a microorganism.

In the above use, the nucleic acid molecule may include any of the followings:
F1) a DNA molecule having a coding sequence as shown in SEQ ID No. 1; and
F2) a DNA molecule having a nucleotide sequence as shown in SEQ ID No. 1.

The present application further provides a use of a nucleic acid molecule that inhibits or reduces the expression of the coding gene of the protein aceA. The use includes any of the followings:
D1) a use in the construction of genetically engineered bacteria that produces L-amino acid;
D2) a use in the preparation of L-amino acid; and
D3) a use in the regulation of the yield of L-amino acid in a microorganism.

In the above use, the nucleic acid molecule may include any of the followings:
H1) sgRNA1, having a target sequence as shown in SEQ ID No. 11; and
H2) sgRNA2, having a target sequence as shown in SEQ ID No. 12.

The present application further provides a use of an expression cassette containing any of the above nucleic acid molecules. The use includes any of the followings:
D1) a use in the construction of genetically engineered bacteria that produces L-amino acid;
D2) a use in the preparation of L-amino acid; and
D3) a use in the regulation of the yield of L-amino acid in a microorganism.

The present application further provides a use of a recombinant vector containing any of the above nucleic acid molecules. The use includes any of the followings:
D1) a use in the construction of genetically engineered bacteria that produces L-amino acid;
D2) a use in the preparation of L-amino acid; and
D3) a use in the regulation of the yield of L-amino acid in a microorganism.

The present application further provides a use of a recombinant microorganism containing any of the above nucleic acid molecules. The use includes any of the followings:
D1) a use in the construction of genetically engineered bacteria that produces L-amino acid;
D2) a use in the preparation of L-amino acid; and
D3) a use in the regulation of the yield of L-amino acid in a microorganism.

The present application further provides a use of a recombinant host cell containing any of the above nucleic acid molecules. The use includes any of the followings:
D1) a use in the construction of genetically engineered bacteria that produces L-amino acid;
D2) a use in the preparation of L-amino acid; and
D3) a use in the regulation of the yield of L-amino acid in a microorganism.

Further, the expression cassette, the recombinant vector, the recombinant microorganism, and the recombinant host cell are capable of expressing any of the above nucleic acid molecules, respectively.

The DNA molecule (named as an aceA gene) as shown in SEQ ID No. 1 codes a protein aceA having an amino acid sequence as shown in SEQ ID No. 2.

The nucleic acid molecule may also include a nucleic acid molecule which is modified by codon preference on the basis of the nucleotide sequence as shown in SEQ ID No. 1.

As used herein, the nucleic acid molecule that inhibits or reduces the expression of the coding gene of the protein aceA may be a nucleic acid molecule that reduces the expression quantity of the aceA gene.

The nucleic acid molecule that inhibits or reduces the expression of the coding gene of the protein aceA may be sgRNA, microRNA, siRNA, shRNA, and/or antisense oligonucleotide.

Further, the sgRNA, microRNA, siRNA, shRNA, and/or antisense oligonucleotide are/is used for inhibiting the expression of the aceA gene.

The sgRNA1 may be used for gene mutation, i.e. for mutation of the aceA gene.

The sgRNA2 may be used for gene knockout, i.e. for knockout of the aceA gene.

It is well known to those skilled in the art that, in addition to using a gene editing technology to inhibit the expression of the aceA gene, a gene knock-down technology may also be used for expression inactivation or gene silencing of the aceA gene at a post-transcriptional or translational level. The gene knock-down technology includes, but is not limited to, RNA interference, Morpholino interference, antisense nucleic acid, ribozyme, or inhibition of dominant-negative mutation. Furthermore, the use of shRNA or siRNA expressed by a virus (e.g., lentivirus or adeno-associated virus) to inhibit the expression of the aceA gene and to silence the aceA gene is also well known to those skilled in the art.

The regulation of the yield of L-amino acid in the microorganism as used herein can either increase (up-regulate) or reduce (down-regulate) the accumulation of L-amino acid in the microorganism (that is, promote or inhibit the biosynthesis of L-amino acid).

The vector as used herein refers to a vector that can transport exogenous DNA or a target gene into a host cell for amplification and expression. The vector may be a cloning vector or an expression vector, including but not limited to: a plasmid, phage (e.g., λ phage or M13 filamentous phage), clay (i.e., cosmid), a Ti plasmid, and a viral vector (e.g., retrovirus (including lentivirus), adenovirus, or adeno-associated virus). In one or more embodiments of the present application, the vector is pET28(a), a pGRB vector, and/or a pREDCas9 plasmid.

The microorganism as used herein may be bacteria, fungus, actinomycetes, protozoa, algae, or viruses. The bacteria may be derived from, but is not limited to: *Escherichia sp., Erwinia sp., Agrobacterium sp., Flavobacterium sp., Alcaligenes sp., Pseudomonas sp., Bacillus sp.,* etc. For example, the bacteria may be *Escherichia coli, Corynebacterium glutamicum, brevibacterium lactofermentum, brevibacterium flavum, Corynebacterium pekinense, Brevibacterium ammoniagenes, Corynebacterium crenatum,* or *Pantoea,* etc. The fungus may be yeast. The yeast may be derived from, but is not limited to: *Saccharomyces sp.* (e.g., *Saccharomyces cerevisiae), Kluyveromyces sp.* (e.g., *Kluyveromyces lactis), Pichia* (e.g., *Pichia pastoris), Schizosaccharomyces, sp.* (e.g., *Schizosaccharomyces pombe), Hansenula (e.g., Hansenula polymorpha).* The fungus may also be derived from, but is not limited to: *Fusarium sp., Rhizoctonia sp., Verticillium sp., Penicillium sp., Aspergillus sp., Cephalosporium sp.,* etc. The actinomycete may be derived from, but is not limited to: *Streptomyces sp., Nocardia sp., Micromonospora sp., Streptosporangium sp., Actinoplanes sp., Thermoactinomyces sp.,* etc. The algae may be derived from, but is not limited to: *Fucus sp., Achnanthes sp., Amphiprora sp., Amphora sp., Ankistrodesmus sp., Asteromonas sp., Boekelovia sp.,* etc. The virus may be, but is not limited to: rotavirus, herpes virus, influenza virus, adenovirus, etc. In one or more embodiments of the present application, the microorganism is *Escherichia coli* DH5α, *Escherichia coli* W3110, and/or *Escherichia coli* CGMCC26289.

The host cell as used herein (also known as a receptor cell) may be a plant cell or an animal cell. The host cell may be understood to refer not only to a specific receptor cell, but also to a descendant of such cell. Due to natural, accidental or intentional mutations and/or alterations, this descendant does not necessarily have to be exactly the same as the original parent cell, but still falls within the scope of the host cell. Suitable host cells are known in the art. The plant cell may be, but is not limited to: *Arabidopsis thaliana,* tobacco *(Nicotiana tabacum),* corn (*Zea mays),* rice *(Oryza sativa),* wheat *(Triticum aestivum)* and other plant cells. The animal cell may be, but is not limited to: mammalian cells (e.g., Chinese hamster ovary cells (CHO cells), African green monkey kidney cells (Vero cells), baby hamster kidney cells (BHK cells), mouse breast cancer cells (C127 cells), human embryonic kidney cells (HEK293 cells), human HeLa cells, fibroblasts, bone marrow cell lines, T cells or NK cells), poultry cells (e.g., chicken or duck cells), amphibian cells (e.g., Xenopus laevis cells or Andrias davidianus cells), fish cells (e.g., grass carp, common carp, rainbow trout or catfish cells), insect cells (e.g., Sf21 cells or Sf-9 cells), etc.

The recombinant vector as used herein refers to a recombinant DNA molecule which is constructed by linking an exogenous target gene or nucleic acid molecule to a vector in vitro.

The recombinant microorganism (or recombinant host cell) as used herein refers to a recombinant microorganism with changed functions (or recombinant host cell with changed functions), which is obtained by performing operation and modification on a gene of a target microorganism (or a target host cell). For example, the recombinant microorganism is a recombinant microorganism (or recombinant host cell) which is obtained by introducing an exogenous target gene or recombinant vector into the target microorganism (or target host cell), or a recombinant microorganism (or recombinant host cell) which is obtained by performing gene editing on an endogenous gene of the target microorganism (or target host cell). The recombinant microorganism (or recombinant host cell) may be understood to refer not only to a specific recombinant microorganism (or recombinant host cell), but also to a descendant of such cell. Due to natural, accidental or intentional mutations and/or alterations, this descendant does not necessarily have to be exactly the same as the original parent cell, but still falls within the scope of the recombinant microorganism (or recombinant host cell).

The recombinant microorganism may be used to produce a variety of products, including but not limited to: alanine, lysine, glutamic acid, valine, glycine, leucine, isoleucine, methionine, proline, tryptophan, serine, tyrosine, cysteine, phenylalanine, asparagine, glutamine, threonine, aspartic acid, arginine, histidine, shikimic acid, protocatechuic acid, succinic acid, α-ketoglutarate, citric acid, ornithine, citrulline, etc.

The recombinant vector as used herein may be pET28(a)-aceA. The recombinant vector pET28(a)-aceA is a recombinant expression vector which is obtained by cloning a wild-type aceA gene and its promoter into an expression vector pET28(a). The recombinant vector pET28(a)-aceA contains an aceA gene as shown in SEQ ID No. 3 and its promoter.

The recombinant microorganism as used herein may be DH5α/ pET28(a)-aceA. The recombinant bacteria DH5α/pET28(a)-aceA is recombinant bacteria which is obtained by introducing the recombinant vector pET28(a)-aceA into *Escherichia coli* DH5α. The recombinant bacteria DH5α/pET28(a)-aceA contains the aceA gene as shown in SEQ ID No. 3 and its promoter.

The present application further provides a method for increasing the yield of L-amino acid in a microorganism. The method may include: reducing the content and/or activity of the protein aceA in a target microorganism to obtain a microorganism with a higher yield of L-amino acid than the target microorganism.

The target microorganism may be a microorganism containing a coding gene of the protein aceA. In the above method, the reducing the content and/or activity of the protein aceA in the target microorganism may be implemented by reducing the expression quantity and/or activity of the coding gene of the protein aceA in the target microorganism.

In the above method, the reducing the expression quantity and/or activity of the coding gene of the protein aceA in the target microorganism may refer to reducing or inactivating the activity of the coding gene of the protein aceA in a genome of the target microorganism by using a gene mutation, gene knockout, gene editing or gene attenuation technology.

In the above method, the reducing or inactivating the activity of the coding gene of the protein aceA in the genome of the target microorganism by using the gene editing technology is performed by using a CRISPR/Cas9 system, wherein the CRISPR/Cas9 system includes a vector that expresses sgRNA targeting the coding gene of the protein; and the sgRNA may include any of the followings:
G1) sgRNA1 for gene mutation, having a target sequence as shown in SEQ ID No. 11; and
G2) sgRNA2 for gene knockout, having a target sequence as shown in SEQ **ID** No. 12.

In the above method, the gene mutation in G1) may be the mutation of a DNA molecule having a nucleotide sequence of SEQ **ID** No. 1 in the target microorganism into a DNA molecule as shown in SEQ ID No. 5.

Specifically, cytosine (C) at position **1111** in SEQ ID No. 1 is mutated into thymine (T) to obtain SEQ ID No. 5.

Further, a point mutation is introduced into the aceA gene coding region (SEQ ID No. 1). The point mutation is to mutate cytosine (C) at position **1111** in the nucleotide sequence (SEQ ID No. 1) of the aceA gene into thymine (T) to obtain the DNA molecule (a mutant aceA gene, named as a mutant aceA^{Q371*} gene) as shown in SEQ **ID** No. 5.

The DNA molecule as shown in SEQ **ID** No. 5 codes a mutant protein (aceA^{Q371*} protein) having an amino acid sequence as shown in SEQ ID No. 6. A termination codon at position 371 in the amino acid sequence (SEQ ID No. 6) of this mutant protein is mutated from glutamine (Q).

The microorganism obtained by any of the methods as used herein or any of the recombinant microorganisms as used herein falls within the protection scope of the present application.

The microorganism obtained by any of the methods as used herein or a use of any of the recombinant microorganism as used herein in the fermentation production of L-amino acid falls within the protection scope of the present application.

The present invention further provides a recombinant microorganism, in which the protein aceA is weakly expressed or not expressed.

Further, the weak expression or no expression is implemented by reducing the expression quantity and/or activity of the coding gene of the protein aceA in the target microorganism.

Further, the reducing the expression quantity and/or activity of the coding gene of the protein aceA in the target microorganism may refer to reducing or inactivating the activity of the coding gene of the protein aceA in a genome of the target microorganism by using a gene mutation, gene knockout, gene editing or gene attenuation technology.

Further, the gene editing may be performed by using a CRISPR/Cas9 system, wherein the CRISPR/Cas9 system includes a vector that expresses sgRNA targeting the coding gene of the protein; and the sgRNA may include any of the followings:
G1) sgRNA1 for gene mutation, having a target sequence as shown in SEQ ID No. 11; and
G2) sgRNA2 for gene knockout, having a target sequence as shown in SEQ ID No. 12.

Further, the gene mutation in G1) may be the mutation of a DNA molecule having a nucleotide sequence of SEQ ID No. 1 in the target microorganism into a DNA molecule as shown in SEQ ID No. 5.

The present invention further provides a recombinant microorganism. The recombinant microorganism contains a nucleic acid molecule that inhibits or reduces the expression of the coding gene of the protein aceA.

Further, the nucleic acid molecule may include any of the followings:
H1) sgRNA1, having a target sequence as shown in SEQ ID No. 11; and
H2) sgRNA2, having a target sequence as shown in SEQ ID No. 12.

The present application further provides a protein named as aceA^{Q371*}. The protein may include any of the followings:
M1) a protein comprising an amino acid sequence as shown in SEQ ID No. 6;
M2) a protein which is obtained by substitution and/or deletion and/or addition of an amino acid residue in the amino acid sequence as shown in SEQ ID No, 6, has the identity of 80% or more with the protein shown in M1) and has the same function as the protein shown in M1); and
M3) a fusion protein which is obtained by linking a tag to an N-terminal or/and a C-terminal of M1) or M2) and has the same function as that of M1) or M2).

The linking in M3) is implemented through a peptide bond.

The present invention further provides a nucleic acid molecule that codes the protein aceA^{Q371*}.

The present invention further provides an expression cassette containing the nucleic acid molecule that codes the protein aceA^{Q371*}.

The present invention further provides a recombinant vector containing the nucleic acid molecule that codes the protein aceA^{Q371*}.

The present invention further provides a recombinant microorganism containing the nucleic acid molecule that codes the protein aceA^{Q371*},

The present invention further provides a recombinant host cell containing the nucleic acid molecule that codes the protein aceA^{Q371*},

Further, the nucleic acid molecule may include the DNA molecule as shown in SEQ ID No. 5.

The present application further provides a use of the protein aceA^{Q371*} or the nucleic acid molecule that codes the protein aceA^{Q371*} in the followings:
N1) a use in the construction of genetically engineered bacteria that produces L-amino acid;
N2) a use in the preparation of L-amino acid; and
N3) a use in the regulation of the yield of L-amino acid in a microorganism.

Thymine (T) at position 1111 of the DNA molecule as shown in SEQ ID No. 5 (which may be named as an aceA^{Q371*} gene) is mutated from cytosine (C), resulting in early termination of translation, ultimately in the final translation of a mutant protein aceA^{Q371*} having an amino acid sequence of SEQ ID No. 6.

The L-amino acid as used herein includes L-alanine, L-valine, L-arginine, L-tryptophan, and/or L-threonine.

Isocitrate lyase (ICL) is an enzyme that cleaves isocitrate into glyoxylic acid and succinic acid in the glyoxylate cycle, with a product being synthesized by malic acid synthase into malic acid. The present application provides a use of an *Escherichia coli* isocitrate lyase gene aceA in the increase of the yield of L-amino acid in a microorganism. In the present application, an aceA gene fragment containing random point mutations, which is obtained through error-prone PCR amplification, is used to construct a random mutant plasmid, followed by transformation of *Escherichia coli* W3110, to obtain aceA gene mutant strains W3110 with different mutations. The concentration (content) of L-alanine is analyzed by HPLC after fermentation culture of the resulting mutant strains. A YPAla-aceA mutant strain 2 was screened out, which has a superior L-alanine production ability than a wild-type W3110 strain and other mutant strains. A sequencing result of an aceA gene in the YPAla-aceA mutant strain 2 indicated that: cytosine (C) at position 1111 in the nucleotide sequence (SEQ ID No. 1) of the aceA gene is mutated into thymine (T), and correspondingly, glutamine (Q) at position 371 in an amino acid sequence (SEQ ID No. 2) of a protein coded by this gene is mutated into a termination codon (the mutated gene is named as an aceA^{Q371*} gene). In order to further study the effects of the aceA gene and the aceA^{Q171*} gene on the yield of L-amino acid in producing bacteria, the following strains are constructed in the present application: 1, an aceA gene mutant engineered strain which is obtained by introducing a point mutation (C-T) to position 1111 of an aceA gene coding region of a high-yield L-amino acid-producing strain and wild-type *Escherichia coli* W3110; and 2, an engineered strain obtained by knocking out an aceA gene from the high-yield L-amino acid-producing strain and the wild-type *Escherichia coli* W3110. The constructed engineered strain is subjected to a fermentation experiment. Results showed that the aceA gene and a variant aceA^{Q171*} gene thereof participate in biosynthesis of L-amino acid, and knocking out or weakening the aceA gene (i.e., inhibiting or reducing the expression of the aceA gene) facilitates accumulation of L-amino acid. The aceA gene and the variant thereof (e.g., the aceA^{Q171*} gene) may be used to construct a genetically engineered strain for producing L-amino acid so as to promote the increase of the yield of L-amino acid.

### Preservation instructions

1. Strain name: *Escherichia coli*
   Latin name: *Escherichia coli*
   Classification name: *Escherichia coli*
   Strain number: YP007-1
   Title of preservation unit: China General Microbiological Culture Collection Center
   Abbreviation of preservation unit: CGMCC
   Address: No. 3, Courtyard 1, Beichen West Road, Chaoyang District, Beijing
   Preservation date: December 26, 2022
   Preservation Center registration number: CGMCC No. 26289
2. Strain name: *Escherichia coli*
   Latin name: *Escherichia coli*
   Classification name: *Escherichia coli*
   Strain number: YP045
   Title of preservation unit: China General Microbiological Culture Collection Center
   Abbreviation of preservation unit: CGMCC
   Address: No. 3, Courtyard 1, Beichen West Road, Chaoyang District, Beijing
   Preservation date: June 15, 2021
   Preservation Center registration number: CGMCC No. 22721
3. Strain name: *Escherichia coli*
   Latin name: *Escherichia coli*
   Classification name: *Escherichia coli*
   Strain number: YP004-8
   Title of preservation unit: China General Microbiological Culture Collection Center
   Abbreviation of preservation unit: CGMCC
   Address: No. 3, Courtyard 1, Beichen West Road, Chaoyang District, Beijing
   Preservation date: July 25, 2022
   Preservation Center registration number: CGMCC No. 25402
4. Strain name: *Escherichia coli*
   Latin name: *Escherichia coli*
   Classification name: *Escherichia coli*
   Strain number: YP006D
   Title of preservation unit: China General Microbiological Culture Collection Center
   Abbreviation of preservation unit: CGMCC
   Address: No. 3, Courtyard 1, Beichen West Road, Chaoyang District, Beijing
   Preservation date: July 25, 2022
   Preservation Center registration number: CGMCC No. 25403
5. Strain name: *Escherichia coli*
   Latin name: *Escherichia coli*
   Classification name: *Escherichia coli*
   Strain number: YP0158
   Title of preservation unit: China General Microbiological Culture Collection Center
   Abbreviation of preservation unit: CGMCC
   Address: No. 3, Courtyard 1, Beichen West Road, Chaoyang District, Beijing
   Preservation date: July 25, 2022
   Preservation Center registration number: CGMCC No. 25404

### Detailed Description of the Invention

The present application is described in further detail below in conjunction with the specific embodiments, and the given examples are only for the purpose of clarifying the present application, but not for the purpose of limiting the scope of the present application. The examples provided below may be used as a guide for further improvement by a person of ordinary skill in the art and do not in any way constitute a limitation of the present application.

The experimental methods in the following examples, unless otherwise specified, are conventional methods and are carried out in accordance with the techniques or conditions described in the literatures in the art or in accordance with the product instructions. The materials, reagents, etc. used in the following examples, unless otherwise specified, may be obtained commercially.

*Escherichia coli* W3110 in the following examples was a strain of the American Type Culture Collection (ATCC) with preservation number of ATCC27325.

A pGRB cloning vector and a pREDCas9 plasmid in the following examples were products from addgene.

L-alanine-producing bacteria CGMCC26289 in the following examples was *Escherichia coli* YP007-1 CGMCC No. 26289, abbreviated as CGMCC26289.

L-valine-producing bacteria CGMCC22721 in the following examples was *Escherichia coli* YP045 CGMCC No. 22721, abbreviated as CGMCC22721.

L-arginine-producing bacteria CGMCC25402 in the following examples was *Escherichia coli* YP004-8 CGMCC No. 25402, abbreviated as CGMCC25402.

L-tryptophan-producing bacteria CGMCC25403 in the following examples was *Escherichia coli* YP006D CGMCC No. 25403, abbreviated as CGMCC25403.

L-threonine-producing bacteria CGMCC25404 in the following examples was *Escherichia coli* YP0158 CGMCC No. 25404, abbreviated as CGMCC25404.

### Example 1: Construction of mutant strains containing isocitrate lyase gene aceA

### I: Construction of mutant plasmids containing isocitrate lyase gene aceA

For ease of study, a wild-type aceA gene (having a nucleotide acid sequence as shown in SEQ ID No. 1) and its promoter were first cloned into an expression vector pET28(a). By taking a genome sequence of *Escherichia coli* W3110 published by NCBI as a template, a wild-type aceA promoter was obtained by performing PCR amplification with primers PaceA-PF and PaceA-PR, and a wild-type aceA gene was obtained by performing PCR amplification with primers aceA-PF and aceA-PR. The recovered product was linked to an expression vector pET28(a) (TaKaRa, containing chloramphenicol resistance) recovered by EcoR I/Hind III enzyme digestion through a NEBuilder enzyme (NEB) at 50°C for 30 min; and the linking product was transformed to *Escherichia coli* DH5α competent cells, coated onto a 2-YT agar plate containing chloramphenicol (50 mg/L), and cultured at 37°C to obtain a pET28(a) transformant DH5α/pET28(a)-aceA containing the aceA gene and its promoter (a nucleotide sequence of the aceA gene and its promoter was shown in SEQ ID No. 3). Monoclones which grew in culture were subjected to PCR identification with primers T7/T7t and r Taq, and those which could produce a fragment of 1889 bp (having a sequence as shown in SEQ ID No. 4) through PCR amplification were a pET28(a) positive transformant pDH5α/pET28(a)-aceA containing aceA and its promoter.

A plasmid was extracted and named as pET28(a)-aceA. The plasmid pET28(a)-aceA (also referred to as a recombinant vector pET28(a)-aceA) was a recombinant expression vector which was obtained by cloning a wild-type aceA gene and its promoter into an expression vector pET28(a). The recombinant vector pET28(a)-aceA contained an aceA gene as shown in SEQ ID No. 3 and its promoter.

The recombinant bacteria DH5α/pET28(a)-aceA was recombinant bacteria which was obtained by introducing the recombinant vector pET28(a)-aceA into *Escherichia coli* DH5α. The recombinant bacteria DH5α/pET28(a)-aceA contained the aceA gene as shown in SEQ ID No. 3 and its promoter.

In order to obtain a mutant that coded an isocitrate lyase gene aceA, a random mutagenesis kit (Agilent Technologies, USA) was used to prepare an aceA mutant gene plasmid (aceA random mutant plasmid). A specific method was as follows: by taking the plasmid pET28(a)-aceA as a template, an aceA gene fragment (1602 bp) containing random point mutations was obtained by performing PCR amplification with primers PaceA-PF and aceA-PR, named as a DNA fragment 1 (having a sequence as shown in SEQ ID No. 3, but there were random point mutations in an aceA coding region).

A PCR amplification system: 10 µL of 5×HiFi with Mg²⁺Buffer, 1.5 µL of dNTP Mixture (10 mM), 1.6 µL of primers (10 pM) each, 0.5 µL of KAPA HiFi HotStart (1U/µL), and ddH₂O supplemented to the total volume of 50 µL.

PCR amplification procedure: pre-denaturation at 95°C for 5 min, (denaturation at 98°C for 20 s; annealing at 56°C for 15 s; extension at 72°C for 60 s, in a total of 30 cycles), and overextension at 72°C for 5 min.

The recovered DNA fragment 1 was linked to an expression vector pET28(a) (TaKaRa, containing chloramphenicol resistance) recovered by EcoR I/Hind III enzyme digestion through an NEBuilder enzyme (NEB) at 50°C for 30 min; and the linking product was transformed to DH5α competent cells, coated onto a 2-YT agar plate containing kanamycin (50 mg/L), and cultured at 37°C. Monoclones which grew in culture were subjected to PCR identification with primers T7/T7t and r Taq, and those which could produce a fragment of 1889 bp (having a sequence as shown in SEQ ID No. 4, but there were random point mutations in an aceA coding region) through PCR amplification were a pET28(a) positive transformant containing aceA random mutations. The extracted plasmid was an aceA random mutant plasmid.

PCR amplification system: 12.5 µL of 2×Premix r Taq, 1 µL of primers (10 pM) each, and ddH₂O supplemented to a total volume of 25 µL.

PCR amplification procedure: pre-denaturation at 94°C for 5 min, denaturation at 94°C for 30 s; annealing at 56°C for 30 s; extension at 72°C for 90 s (in a total of 30 cycles), and overextension at 72°C for 10 min.

The primers were designed as follows (synthesized by Shanghai Invitrogen Company):
PaceA-PF: 5'-TAGCATGACTGGTGGACAGCAAATGGGTCGCGGATCCTCTTCTGTGATAGTCGATCG-3' (the underlined nucleotide sequence was a pET28(a) homologous arm sequence) (SEQ ID No. 13),
PaceA-PR: 5'-CTCCATAGTTATGTGGTGGTCGTGCAGCTCCTCGTCATG-3' (SEQ ID No. 14),
aceA-PF: 5'-CCATGACGAGGAGCTGCACGACCACCACATAACTATGGAG-3' (SEQ ID No. 15),
aceA-PR: 5'-CGGATCTCAGTGGTGGTGGTGGTGGTGCTCGAGTGCTTAGAACTGCGATTCTTCAGTG-3' (the underlined nucleotide sequence was a pET28(a) homologous arm sequence) (SEQ ID No. 16),
T7: 5'-TAATACGACTCACTATAGGG-3' (SEQ ID No.17), and
T7t: 5'-GCTAGTTATT GCTCAGCGG-3' (SEQ ID No. 18).

### II: Construction of mutant strains containing isocitrate lyase gene aceA

To identify the L-alanine production performance of the mutant vector constructed in step I, specifically, the aceA random mutant plasmid constructed in step I was transformed into an alanine-producing *Escherichia coli* CGMCC26289 strain (same as step I for transformation and identification); a positive transformant was passaged three times in a 2-YT agar plate containing kanamycin (50 mg/L), inoculated into a 500 mL triangular flask filled with 30 mL of rich medium and fermented at 37°C for 24 h under shaking; and after the fermented culture bacteria grew to OD₆₀₀=0.2, IPTG having a final concentration of 0.1 mM was added to induce overexpression of isocitrate lyase.

After the fermentation culture, the concentration of L-amino acid was analyzed by high-performance liquid chromatography (HPLC), as shown in Table 1. A strain with superior L-alanine-producing capacity, i.e., a YPAla-aceA mutant strain, was picked out.

Rich medium: water was used as a solvent; solute and its concentration were 30 g/L glucose, 2 g/L (NH4)₂SO₄, 0.5 g/L H₃PO₄, 0.8 g/L KCl, 0.8 g/L MgSO₄ . 7H₂O, 0.05 g/L FeSO₄ . 7H₂O, 0.05 g/L MnSO₄ . H₂O, 1.5 g/L FM902 yeast powder, 1.5 g/L corn syrup, 17 g/L molasses, 0.5 g/L betaine, 2 g/L citric acid, 20 mg/L VH, 1.5 mg/L VB₁, 1.5 mg/L VB₃, 1.5 g/L VB₁₂, and sodium hydroxide (for adjusting pH to 7.0).

**Table 1: High-performance liquid chromatography analysis results of L-alanine of YPAla-aceA mutant strains**

| Strain Nos. | CGMCC26289 | YPAla-ace A mutant strain 1 | YPAla-ace A mutant strain 2 | YPAla-ace A mutant strain 3 | YPAla-ace A mutant strain 4 | YPAla-ace A mutant strain 5 |
|---|---|---|---|---|---|---|
| L-alanine g/L | 97.200 | 94.420 | 97.080 | 95.040 | 94.830 | 93.980 |

As shown in Table 1, the *Escherichia coli* YPAla-aceA mutant strain disclosed in the present application had the ability to produce L-alanine, wherein the YPAla-aceA mutant strain 2 had a superior ability to produce L-alanine, which was basically the same as that of producing bacteria CGMCC26289.

Based on a sequencing result of the aceA gene by extracting a plasmid from the YPAla-aceA mutant strain 2, it was confirmed that: cytosine (C) at position 1111 in a nucleotide sequence of an aceA mutant was mutated into thymine (T), and glutamine (Q) at position 371 in an amino acid sequence of a mutant protein aceA was mutated into a termination codon. This plasmid was pET28(a)-aceA^{Q371*} (having a sequence as shown in SEQ ID No. 3), and its mutation at position 1408 was (T). The DNA sequence as shown in SEQ ID No. 1 was a wild-type aceA gene, and an amino acid sequence of a coding protein was SEQ ID No. 2 (this protein was named as a wild-type aceA protein). The DNA sequence as shown in SEQ ID No. 5 was a mutant aceA^{Q371*} gene, thymine (T) at position 1111 in the mutant aceA^{Q371*} gene sequence (SEQ ID No. 5) was mutated from cytosine (C), the amino acid sequence of the coding protein was SEQ ID No. 6 (the mutant protein was named as a mutant aceA^{Q371*} protein), and a termination codon at position 371 in the amino acid sequence (SEQ ID No. 6) of the mutant protein aceA^{Q371*} was mutated from glutamine (Q).

### Example 2: Construction of mutant engineered strains containing isocitrate lyase gene aceA

According to the genome sequence of *Escherichia coli* W3110 published by NCBI, a CRISPR/Cas9 gene editing technology was used to perform point mutation on an aceA gene of a high-yield L-amino acid-producing strain and wild-type *Escherichia coli* W3110. In this way, a more in-depth study was conducted on the aceA gene and the mutant aceA^{Q371*} gene in the high-yield strain, to investigate their effects on the yields of amino acids such as L-alanine, L-valine, L-arginine, L-tryptophan, and L-threonine.

A point mutation was introduced into an aceA gene coding region (SEQ ID No. 1). The point mutation was to mutate cytosine (G) at position 1111 in the nucleotide sequence (SEQ ID No. 1) of the aceA gene into thymine (T) to obtain the DNA molecule (a mutant aceA gene, named as a mutant aceA^{Q371*} gene) as shown in SEQ ID No. 5.

Correspondingly, the DNA molecule as shown in SEQ ID No. 5 coded a mutant protein (aceA^{Q371*} protein) having an amino acid sequence as shown in SEQ ID No. 6. A termination codon at position 371 in the amino acid sequence (SEQ ID No. 6) of this mutant protein was mutated from glutamine (Q).

### I. Construction of sgRNA

According to the genome sequence of *Escherichia coli* W3110 published by NCBI, an sgRNA 00(sgRNA1) target sequence was designed by using CRISPR RGEN Tools (http://www.rgenome.net/cas-designer/), and after an appropriate sgRNA target sequence was selected, a linearized pGRB cloning vector terminal sequence was added to ends 5' and 3' of the target sequence, in order to form a complete sgRNA plasmid through recombination.

The target sequence of sgRNA1 was follows: 5'-AGCGGCGGACGCTAACCTGGCGG-3' (SEQ ID No. 11); and sgRNA1 was used to mutate a base C at position 1111 of the aceA gene into a base T to obtain a mutant gene (the aceA^{Q371*} gene) and a mutant protein (the aceA^{Q371*} protein).

A DNA fragment containing the sgRNA1 target sequence was amplified without a template, just by performing a PCR annealing process. The system and procedure were as follows. PCR reaction system: 10 µL of sgRNA-1F, and 10 µL of sgRNA-1R; and PCR reaction procedure: denaturation at 95°C for 5 min, and annealing at 50°C for 1 min. After annealing, the DNA concentration of the target fragment (a DNA fragment containing the sgRNA1 target sequence) was determined by using a DNA purification kit, and the concentration was diluted to 100 ng/µL.

The pGRB plasmid was subjected to Spe I enzyme digestion and dephosphorylation to prevent self-adhesion of the pGRB plasmid. Enzyme digestion system: 5 µL of 10×Buffer, 2.5 µL of Spe I, 3000-5000 ng of pGRB plasmid DNA, and ddH₂O supplemented to 50 µL. After 3 h of enzyme digestion at 37°C, agarose gel electrophoresis was performed for gel cutting and recovery, followed by a dephosphorylation reaction. The dephosphorylation system included: 5 µL of 10×Buffer, 1000-2000 ng of linearized pGRB plasmid DNA, 2.5 µL of CIAP, and ddH₂O supplemented to 50 µL. After being treated at 37°C for 1 h, the linearized pGRB plasmid was recovered using a DNA purification kit. A Gibson Assembly Kit (New England) was then used to recombine a DNA fragment containing an sgRNA1 target sequence and the linearized pGRB plasmid. Recombination system: 2.5 µL of NEB-assembling enzyme, 2 µL of linearized pGRB plasmid, and 0.5 µL of DNA fragment containing the sgRNA1 target sequence. After 30 min of assembly at 50°C, the product was transformed into DH5α competent cells; and plasmids were extracted, followed by sequencing and identification with sequencing primers sgRNA-PF/sgRNA-PR. The constructed plasmid was named as pGRB-sgRNA-1.

The primers used in this experiment were designed as follows (synthesized by Shanghai Invitrogen Company), with the underlined base being a homologous arm sequence of a pGRB cloning vector and the bolded base being the sgRNA1 target sequence:
sgRNA-1F: 
5'-TGACAGCTAGCTCAGTCCTAGGTATAATACTAGTAGCG
GCGGACGCTAACCTGGCGGGTTTTAGAGCTAGAAATAGCAAGTTAAAAT
AAGG-3'（SEQ ID No.19），
sgRNA-1R: 
5'-CCTTATTTTAACTTGCTATTTCTAGCTCTAAAACCCGCC
AGGTTAGCGTCCGCCGCTACTAGTATTATACCTAGGACTGAGCTAGCTGT
CA-3'（SEQ ID No.20），
sgRNA-PF: 5'-GTCTCATGAGCGGATACATATTTG-3'(SEQ ID No. 21), sgRNA-PR: 5'-ATGAGAAAGCGCCACGCT-3'(SEQ ID No. 22).

### II. Amplification of genetically mutated aceA^{Q371*} DNA

With a W3110 genome DNA as a template, two aceA^{Q371*} DNA fragments (aceA ^{Q371}*Up and aceA ^{Q371*}Down) with mutant base sizes of 641 bp and 628 bp respectively were obtained by performing PCR amplification with primers P1/P2, P3/P4 and KAPA HiFi HotStart, respectively. After the PCR reaction, agarose gel electrophoresis was performed by a column DNA gel recovery kit to recover aceA^{Q371*}Up and aceA^{Q371}*Down, respectively. By taking two recovered DNAs as a template, overlap PCR amplification was performed with primers P1/P4 to obtain a DNA fragment Up-aceA^{Q371}*-Down (SEQ ID No. 7) (1241 bp) of a point mutation-integrated homologous arm. A PCR amplification system: 10 µL of 5×HiFi with Mg²⁺Buffer, 1.5 µL of dNTP Mixture (10 mM), 1.6 µL of primers (10 pM) each, 0.5 µL of KAPA HiFi HotStart (1U/µL), and ddH₂O supplemented to the total volume of 50 µL.

PCR amplification procedure: pre-denaturation at 95°C for 5 min, (denaturation at 98°C for 20 s; annealing at 56°C for 15 s; extension at 72°C for 60 s, in a total of 30 cycles), and overextension at 72°C for 5 min.

The primers were designed as follows (synthesized by Shanghai Invitrogen Company), and a base of a lowercase bold font was a mutation site:
P1: 5'-TTGGCG GTGTCCTGAATGCC-3' (SEQ ID No. 23),
P2:
   5'-CATACCCTCGCCCTaGGCATAGGCGTTTGCCAGGTC-3' (SEQ ID No.24) ,
P3:
   5'-AAACGCCTATGCCtAGGGCGAGGGTATGAAGCAC-3' (SEQ ID No.25) ,
P4: 5'-GTAAAAATGCCGCGTCCGTAC-3' (SEQ ID No. 26).

### III. Preparation and transformation of competent cells

The pREDCas9 plasmid (containing a spectinomycin-resistant gene) was transformed into L-alanine-producing bacteria CGMCC26289, L-valine-producing bacteria CGMCC22721, L-arginine-producing bacteria CGMCC25402, L-tryptophan-producing bacteria CGMCC25403, L-threonine-producing bacteria CGMCC25404 and wild-type *Escherichia coli* W3110 competent cells, respectively, coated to a 2-YT agar plate containing spectinomycin (100 mg/L), and cultured at 32°C. Single colonies resistant to spectinomycin (100 mg/L) were subjected to PCR identification with primers pRedCas9-PF/pRedCas9-PR by using r Taq. The colonies containing a fragment of 943 bp (SEQ ID No. 8) were obtained as L-alanine-producing bacteria CGMCC26289-Cas9, L-valine-producing bacteria CGMCC22721-Cas9, L-arginine-producing bacteria CGMCC25402-Cas9, L-tryptophan-producing bacteria CGMCC25403-Cas9, L-threonine-producing bacteria CGMCC25404-Cas9 and wild-type *Escherichia coli* W3110-Cas9, which contained the pREDCas9 plasmid.

L-alanine-producing bacteria CGMCC26289-Cas9, L-valine-producing bacteria CGMCC22721-Cas9, L-arginine-producing bacteria CGMCC25402-Cas9, L-tryptophan-producing bacteria CGMCC25403-Cas9, L-threonine-producing bacteria CGMCC25404-Cas9 and wild-type *Escherichia coli* W3110-Cas9 competent cells were prepared. When the bacteria grew to OD₆₀₀=0.1, IPTG having a final concentration of 0.1 mM was added to induce λ-Red-mediated homologous recombination. When OD₆₀₀=0.6, the bacteria was collected, and competent cells were prepared, transformed into pGRB-sgRNA-1 plasmids and point mutant recombinant DNA fragment Up-aceA^{Q171*}-Down, coated to a 2-YT agar plate containing spectinomycin (100 mg/L) and ampicillin (100 mg/L), and cultured at 32°C. The transformant was subjected to PCR amplification with primers P5/P6 and r Taq. The obtained PCR product (280 bp) (SEQ ID No.9) was denaturated at 95°C for 10 min, and treated in an ice bath for 5 min, followed by SSCP (Single-Strand Conformation Polymorphis) electrophoresis (with Up-aceA^{Q371*}-Down amplified PCR fragment as a positive control, W3110 amplified PCR fragment as a negative control and water as a blank control). Due to different fragment structures and different electrophoresis positions, the strains whose electrophoresis position of the PCR fragment was inconsistent with the position of a negative control fragment but consistent with the position of a positive control fragment were strains which were successfully undergone allelic substitution.

The primers were designed as follows (synthesized by Shanghai Invitrogen Company):
P5: 5'-GGCAGAAAAACCTCGACGAC-3' (SEQ ID No. 27),
P6: 5'-CCCTGAATAATAGTCGTCAC-3' (SEQ ID No. 28),
pRedCas9-PF: 5'-GCAGTGGCGGTTTTCATG-3' (SEQ ID No. 29),
pRedCas9-PR: 5'-CCTTGGTGATCTCGCCTTTC-3' (SEQ ID No. 30).

PCR amplification system: 12.5 µL of 2×Premix r Taq, 1 µL of primers (10 pM) each, and ddH₂O supplemented to a total volume of 25 µL.

PCR amplification procedure: pre-denaturation at 94°C for 5 min, denaturation at 94°C for 30 s; annealing at 56°C for 30 s; extension at 72°C for 90 s, in a total of 30 cycles), and overextension at 72°C for 10 min.

Preparation and electrophoresis conditions for SSCP electrophoresis PAGE: 8 mL of 40% acrylamide, 4 mL of glycerol, 2 mL of 10×TBE, 40 µL of TEMED, 600 µL of 10% APS, 26 mL of ddH₂O; and an electrophoresis tank was placed in ice water, applied with a voltage of 120 V, and subjected to electrophoresis in 1×TBE buffer for 10 h.

Transformants which undergone successful point mutation were inoculated into a 2-YT medium containing spectinomycin (100 mg/L) and arabinose (having a final concentration of 0.2%) to eliminate a plasmid pGRB-sgRNA-1. Colonies that grew on spectinomycin (100 mg/L) but not on ampicillin (100 mg/L) were picked out. These colonies were then transferred to the 2-YT medium, and cultured at 42°C to eliminate pREDCas9 plasmids. Colonies that did not grow on spectinomycin (100 mg/L) but on antibody-free 2-YT were picked out, and then sequenced and identified again for a PCR amplification point mutation sequence by using primers P5/P6. A sequencing result was compared with a genome sequence of W3110, and those in which a base C at position 1111 of the aceA gene was mutated into a base T was determined as a gene mutant aceA^{Q371*} positive transformant. An L-alanine-producing strain of the mutant aceA^{Q371*} was named as YPAla-aceA001; an L-valine-producing strain of the mutant aceA^{Q371*} was named as YPV-aceA001; an L-arginine-producing strain of the mutant aceA^{Q371*} was named as YPR-aceA001; an L-tryptophan-producing strain of the mutant aceA^{Q371*} was named as YPTrp-aceA001; an L-threonine-producing strain of the mutant aceA^{Q371*} was named as YPThr-aceA001; and an *Escherichia coli* W3110 of the mutant aceA^{Q371*} was named as W3110-aceA001.

### Example 3: Engineered strains with isocitrate lyase gene aceA missing on the genome

According to the genome sequence of *Escherichia coli* W3110 published by NCBI, a CRISPR/Cas9 gene editing technology was used to knock out the aceA gene from L-alanine-producing bacteria CGMCC26289, L-valine-producing bacteria CGMCC22721, L-arginine-producing bacteria CGMCC25402, L-tryptophan-producing bacteria CGMCC25403, L-threonine-producing bacteria CGMCC25404 (after sequencing, it was confirmed that these amino acid-producing strains retained a wild-type aceA gene on their chromosomes) and wild-type *Escherichia coli* W3110, and the effects of the *Escherichia coli* aceA gene on the synthesis of L-alanine, L-valine, L-arginine, L-tryptophan, L-threonine and other amino acids were then studied.

### I. Construction of sgRNA

According to the genome sequence of *Escherichia coli* W3110 published by NCBI, an sgRNA (sgRNA2) target sequence was designed by using CRISPR RGEN Tools (http://www.rgenome.net/cas-designer/), and after an appropriate sgRNA target sequence was selected, a linearized pGRB cloning vector homologous arm sequence was added to ends 5' and 3' of the target sequence, in order to form a complete sgRNA plasmid through recombination.

The target sequence of sgRNA2 was: 5'-CCTGGCGGCCAGCATGTATCCGG-3' (SEQ ID No. 12); sgRNA2 was used to knock out the aceA gene.

A DNA fragment containing the sgRNA2 target sequence was amplified without a template, just by performing a PCR annealing process; and the system and procedure were as follows: a PCR reaction system: 10 µL of sgRNA-3F, and 10 µL of sgRNA-3R; and PCR reaction procedure: denaturation at 95°C for 5 min, and annealing at 50°C for 1 min. After annealing, the DNA concentration of the target fragment (a DNA fragment containing the sgRNA2 target sequence) was determined by using a DNA purification kit, and the concentration was diluted to 100 ng/µL. The pGRB plasmid was subjected to Spe I enzyme digestion and dephosphorylation to prevent self-adhesion of the pGRB plasmid. Enzyme digestion system: 5 µL of 10×Buffer, 2.5 µL of Spe I, 3000-5000 ng of pGRB plasmid DNA, and ddH₂O supplemented to 50 µL. After 3 h of enzyme digestion at 37°C, agarose gel electrophoresis was performed for gel cutting and recovery, followed by a dephosphorylation reaction. The dephosphorylation system included: 5 µL of 10×Buffer, 1000-2000 ng of linearized pGRB plasmid DNA, 2.5 µL of CIAP, and ddH₂O supplemented to 50 µL. After being treated at 37°C for 1 h, the linearized pGRB plasmid was recovered using a DNA purification kit. A Gibson Assembly Kit (New England) was then used to recombine a DNA fragment containing an sgRNA2 target sequence and the linearized pGRB plasmid. Recombination system: 2.5 µL of NEB-assembling enzyme, 2 µL of linearized pGRB plasmid, and 0.5 µL of DNA fragment containing the sgRNA2 target sequence. After 30 min of assembly at 50°C, the product was transformed into DH5α competent cells; and plasmids were extracted, followed by sequencing and identification with sequencing primers sgRNA-PF/sgRNA-PR. The constructed plasmid was named as pGRB-sgRNA-2.

The primers used in this experiment were designed as follows (synthesized by Shanghai Invitrogen Company), with the underlined base being a homologous arm sequence of a pGRB cloning vector and the base in red font is being a sgRNA2 target sequence:
sgRNA-2F: 
5'-TGACAGCTAGCTCAGTCCTAGGTATAATACTAGTCCTGG
CGGCCAGCATGTATCCGGGTTTTAGAGCTAGAAATAGCAAGTTAAAATA
AGG-3'（SEQ ID No.31），
sgRNA-2R: 
5'-CCTTATTTTAACTTGCTATTTCTAGCTCTAAAACCCGGA
TACATGCTGGCCGCCAGGACTAGTATTATACCTAGGACTGAGCTAGCTGT
CA-3'（SEQ ID No.32），
sgRNA-PF: 5'-GTCTCATGAGCGGATACATATTTG-3' (SEQ ID No. 21), sgRNA-PR: 5'-ATGAGAAAGCGCCACGCT-3' (SEQ ID No. 22).

### II. PCR amplification of recombinant DNA fragment missing on the genome

According to the genome sequence of *Escherichia coli* W3110 published by NCBI, two pairs of primers for amplifying upstream and downstream homologous arm sequences were designed and synthesized; and an aceA gene was knocked from L-alanine-producing bacteria CGMCC26289, L-valine-producing bacteria CGMCC22721, L-arginine-producing bacteria CGMCC25402, L-tryptophan-producing bacteria CGMCC25403, L-threonine-producing bacteria CGMCC25404 and wild-type *Escherichia coli* W3110 by using a CRISPR/Cas9 gene editing method.

The primers were designed as follows (synthesized by Shanghai Invitrogen Company):
P7: 5'-GATGAAGAGCACAATAACCAGG-3' (SEQ ID No. 33),
P8: 5'-CAGTCAGCAACGGTTGTTGTTGCGTGCAGATGCTCCATAGTTAT G-3' (SEQ ID No. 34),
P9: 5'-CATAACTATGGAGCATCTGCACGCAACAACAACCGTTGCTGAC TG-3' (SEQ ID No. 35),
P10: 5'-CGCATCAGTAGAGATTCCCAGC-3' (SEQ ID No. 36).

By taking W3110 genome DNA as a template, PCR amplification was performed with primers P7/P8, P9/P10 and KAPA HiFi Hot Start to obtain upper and lower homologous arm fragments having sizes of 643 bp and 668 bp, respectively. After the PCR reaction, agarose gel electrophoresis recovery was performed by a column DNA gel recovery kit. The recovered DNA was subjected to overlap PCR amplification with primers P7/P10 to obtain a recombinant DNA fragment ΔaceA-Up-Dwon (SEQ ID No. 10) (1266 bp) with an aceA gene missing on the genome. A PCR amplification system: 10 µL of 5×HiFi with Mg²⁺Buffer, 1.5 µL of dNTP Mixture (10 mM), 1.6 µL of primers (10 pM) each, 0.5 µL of KAPA HiFi HotStart (1U/µL), and ddH₂O supplemented to the total volume of 50 µL.

PCR amplification procedure: pre-denaturation at 95°C for 5 min, (denaturation at 98°C for 20 s; annealing at 56°C for 15 s; extension at 72°C for 60 s, in a total of 30 cycles), and overextension at 72°C for 5 min.

### III. Preparation and transformation of competent cells

Competent cells of L-alanine-producing bacteria CGMCC26289-Cas9, L-valine-producing bacteria CGMCC22721-Cas9, L-arginine-producing bacteria CGMCC25402-Cas9, L-tryptophan-producing bacteria CGMCC25403-Cas9, L-threonine-producing bacteria CGMCC25404-Cas9 and wild-type *Escherichia coli* W3110-Cas9 were prepared. When the bacteria grew to OD₆₀₀=0.1, IPTG having a final concentration of 0.1 mM was added to induce λ-Red-mediated homologous recombination. When OD₆₀₀=0.6, the bacteria was collected, and the competent cells were prepared, respectively transformed into pGRB-sgRNA-2 plasmids and recombinant DNA fragment ΔaceA-Up-Dwon with aceA missing on the genome, coated to a 2-YT agar plate containing spectinomycin (100 mg/L) and ampicillin (100 mg/L), and cultured at 32°C. Single colonies produced by culture were subjected to PCR identification with primers P7/P10 by using r Taq, wherein the ones that could produce a fragment of 1266 bp (SEQ ID No. 10) through PCR amplification were positive transformants, while the ones that could produce a fragment of 2571 bp through amplification were probiotics.

The positive transformants were inoculated into a 2-YT medium containing spectinomycin (100 mg/L) and arabinose (having a final concentration of 0.2%) to eliminate a plasmid pGRB-sgRNA-2. Colonies that grew on spectinomycin (100 mg/L) but not on ampicillin (100 mg/L) were picked out. These colonies were then transferred to the 2-YT medium, and cultured at 42°C to eliminate pREDCas9 plasmids. Colonies that did not grow on spectinomycin (100 mg/L) but on antibody-free 2-YT were picked out, and then subjected to PCR identification again with primers P7/P10 by using r Taq, and the ones that could produce a fragment of 1266 bp (SEQ ID No. 10) through PCR amplification were positive transformants.

The positive transformant with the aceA gene missing on a genome of L-alanine-producing bacteria CGMCC26289 was named YPAla-aceA002; the positive transformant with the aceA gene missing on a genome of L-valine-producing bacteria CGMCC22721 was named as YPV-aceA002; the positive transformant with the aceA gene missing on a genome of L-arginine-producing bacteria CGMCC25402 was named as YPR-aceA002; the positive transformant with the aceA gene missing on a genome of L-tryptophan-producing bacteria CGMCC25403 was named as YPTrp-aceA002; the positive transformant with the aceA gene missing on a genome of L-threonine-producing bacteria CGMCC25404 was named as YPThr-aceA002; and the positive transformant with the aceA gene missing on the genome of *Escherichia coli* W3110 was named as W3110-aceA002.

PCR amplification system: 12.5 µL of 2×Premix r Taq, 1 µL of primers (10 pM) each, and ddH₂O supplemented to a total volume of 25 µL.

PCR amplification procedure: pre-denaturation at 94°C for 5 min, denaturation at 94°C for 30 s; annealing at 56°C for 30 s; extension at 72°C for 90 s (in a total of 30 cycles), and overextension at 72°C for 10 min.

### Example 4: Fermentation experiment

### I. Alanine fermentation experiment

The *Escherichia coli* W3110 strain, L-alanine-producing bacteria CGMCC26289 and aceA engineered strains YPAla-aceA001, W3110-aceA001, YPAla-aceA002 and W3110-aceA002 were inoculated in a 5 L fermentation tank of BLBIO-5GC-4-H model (Shanghai Bailun Biotechnology Co., Ltd.), respectively. The fermentation experiment was carried out with an L-alanine fermentation medium according to culture conditions. This process was repeated three times for each strain. After the fermentation, the content of L-alanine was detected by high-performance liquid chromatography (HPLC). The results were averaged from three replicates, as shown in Table 2.

L-alanine fermentation medium: water was used as a solvent; solute and its concentration were 13 g/L glucose, 1 g/L (NH4)₂SO₄, 0.5 g/L H₃PO₄, 0.8 g/L KCl, 0.8 g/L MgSO₄ . 7H₂O, 0.01g/L FeSO₄ . 7H₂O, 0.01g/L MnSO₄ . H₂O, 1.5 g/L FM902 yeast powder, 1.5 g/L corn syrup, 17 g/L molasses, and ammonia introduced to adjust pH to 7.0.

L-alanine fermentation culture conditions:
corrected DO of 100%, temperature of 37°C, air volume of 5 L/min, speed of 800 rpm, tank pressure of 0 Mpa, and calibrated after 5min;
inoculation amount: 10%;
initial conditions: pH of 7.0, culture temperature of 37°C, tank pressure of 0 Mpa, air volume of 0.5 L/min, and speed of 400 rpm;
whole-process control: 1, the dissolved oxygen < 30%, and the speed was sequentially increased to 500 rpm-600 rpm→ air volume of 1 L/min→700 rpm→800 rpm; 2, the tank pressure was increased to 0.01 Mpa after 8 h of fermentation, and the tank pressure was increased to 0.02 Mpa-0.03 Mpa→ 0.04 Mpa-0.05 Mpa after 12 h of fermentation;
residual sugar control: 0.1% -0.5% before F12h; 0.1% -0.3% after F12h in combination with DO requirements;
material fed-batch: 25% of ammonia water, 55% of concentrated sugar, and 10% of Paodi; and
fermentation cycle: about 30 h; and 20% -30% of dissolved oxygen was used in the control process as a standard for increasing and decreasing the air volume.

**Table 2: Yields of L-alanine**

| Strains | L-alanine concentration-1 (g/L) | L-alanine concentration-2 (g/L) | L-alanine concentration-3 (g/L) | Mean (g/L) | P-value |
|---|---|---|---|---|---|
| *Escherichia coli* | 0.009 | 0.007 | 0.007 | 0.008 | |
| W3110-aceA001 | 0.147 | 0.152 | 0.132 | 0.144 | P<0.01 |
| W3110-aceA002 | 0.107 | 0.112 | 0.094 | 0.104 | P<0.01 |
| CGMCC26289 | 97.44 | 97.58 | 96.83 | 97.28 | |
| YPAla-aceA001 | 102.39 | 100.36 | 101.18 | 101.31 | P<0.01 |
| YPAla-aceA002 | 100.44 | 99.53 | 98.68 | 99.55 | P<0.01 |

The above fermentation results indicated that both for a high-yield L-alanine-producing strain CGMCC26289 and wild-type *Escherichia coli* W3110, glutamine at position 371 in the amino acid sequence of the aceA gene was replaced by a termination codon or the aceA gene was completely knocked out, both of which were conducive to the increase in the yield of L-alanine.

### II. L-valine fermentation experiment

The *Escherichia coli* W3110 strain, L-valine-producing bacteria CGMCC22721 and aceA engineered strains YPV-aceA001, W3110-aceA001, YPV-aceA002 and W3110-aceA002 were inoculated in a 5 L fermentation tank of BLBIO-5GC-4-H model (Shanghai Bailun Biotechnology Co., Ltd.), respectively. The fermentation experiment was carried out with an L-valine fermentation medium according to culture conditions. This process was repeated three times for each strain. After the fermentation, the content of L-valine was detected by high-performance liquid chromatography (HPLC). The results were averaged from three replicates, as shown in Table 3.

L-valine fermentation medium: water was used a solvent; and solute and its concentration were: 4 g/L yeast extract powder, 2 g/L corn pulp dry powder, 4 g/L peptone, 2 g/L methionine, 7 g/L KH₂PO₄·3H₂O, 2 g/L MgSO₄·7H₂O, 20 mg/L CoCl₂, 3 g/L (NH4)₂SO₄, 2 g/L citric acid, 50 mg/L FeSO₄·7H₂O, 30 mg/L MnSO₄·7H₂O, 20 mg/L VH, 1.5 mg/L VB₁, 1.5 mg/L VB₃, 1.5 g/L VB₁₂, 0.3 mL/L defoamer, and 3 g/L (NH₄)₂SO₄ (for adjusting pH to 7.0).

L-valine fermentation and culture conditions: dissolved oxygen electrode calibration method: a zero point was calibrated in a saturated sodium sulfite solution and 100% point was calibrated in air; and

L-valine fermentation included two stages of aerobic fermentation and oxygen-restricted fermentation; the cells were first cultured under aerobic fermentation; the air volume, speed and sugar supplementation rate were adjusted in the early stage to control the dissolved oxygen at about 25%; when the OD₆₀₀ value reached 50-60, the speed dropped to 400 rpm and the air volume dropped to 2 L/min; and the aerobic fermentation was changed to oxygen-restricted fermentation.

Corrected DO of 100%, temperature of 33°C, air volume of 1 L/min, speed of 400 rpm, tank pressure of 0.01 Mpa, and calibrated after 5 min;
inoculation amount: 3.5%;
fermentation cycle: about 18-20 h.

**Table 3: Yields of L-valine**

| Strains | L-valine | L-valine | L-valine | Mean | P-value |
|---|---|---|---|---|---|
| *Escherichia coli* W3110 | 0.009 | 0.005 | 0.006 | 0.007 | |
| W3110-aceA001 | 0.105 | 0.096 | 0.095 | 0.099 | P<0.01 |
| W3110-aceA002 | 0.095 | 0.116 | 0.093 | 0.101 | P<0.01 |
| CGMCC22721 | 78.11 | 78.48 | 78.49 | 78.360 | |
| YPV-aceA001 | 79.89 | 79.98 | 79.73 | 79.867 | P<0.01 |
| YPV-aceA002 | 79.69 | 79.6 | 79.99 | 79.760 | P<0.01 |

The above fermentation results indicated that both for a high-yield L-valine-producing strain CGMCC22721 and wild-type *Escherichia coli* W3110, glutamine at position 371 in the amino acid sequence of the aceA gene was replaced by a termination codon or the aceA gene was completely knocked out, both of which were conducive to the increase in the yield of L-valine.

### III. L-arginine fermentation experiment

The *Escherichia coli* W3110 strain, L-arginine-producing bacteria CGMCC25402 and aceA engineered strains YPR-aceA001, W3110-aceA001, YPR-aceA002 and W3110-aceA002 were inoculated in a 5 L fermentation tank of BLBIO-5GC-4-H model (Shanghai Bailun Biotechnology Co., Ltd.), respectively. The fermentation experiment was carried out with an L-arginine fermentation medium according to culture conditions. This process was repeated three times for each strain. After the fermentation, the content of L-arginine was detected by high-performance liquid chromatography (HPLC). The results were averaged from three replicates, as shown in Table 4.

L-arginine fermentation medium: water was used as a solvent; solute and its concentration were 8 g/L glucose, 3 g/L FM902 yeast powder, 6 g/L K₂HPO₄·3H₂O, 1 g/L MgSO₄·7H₂O, 0.05 g/L FeSO₄·7H₂O, 0.5 g/L betaine, 0.005 g/L VB₁₂, 0.3 mL/L defoamer, and 3 g/L ammonium sulfate (adjusting pH to 7.2).

L-arginine fermentation and culture conditions: corrected to DO of 100%: temperature of 35°C, pH of 7.2, speed of 100 rpm, air volume of 6 L/min, and tank pressure of 0.00 Mpa;
inoculation amount: 10%;
initial conditions: temperature of 35°C, pH of 7.2, tank pressure of 0.01 Mpa, air volume of 1.5 L/min, and speed of 350 rpm;
whole-process control: DO was controlled to 20%-30%; when the dissolved oxygen was less than or equal to 25%, the speed was sequentially increased to 300 rpm→400 rpm→2.0 L/min→500 rpm→0.02 Mpa→600 rpm-3.0 L/min→0.03 Mpa→700 rpm-3.5 L/min-0.04 Mpa-800 rpm→900 rpm→4.0 L/min-0.05 Mpa→1000rpm;
residual sugar control: residual sugar was controlled to 0.05%-0.1% throughout the whole process; material fed-batch: 25% of ammonia water, 80% of concentrated sugar, and 10% of Paodi; and Fermentation cycle: about 50 h; and 20%-30% of dissolved oxygen was used in the control process as a standard for increasing and decreasing the air volume.

**Table 4: Yields of L-arginine**

| Strains | L-arginine concentration- | L-arginine concentration- | L-arginine concentration-3 | Mean (g/L) | P-value |
|---|---|---|---|---|---|
| *Escherichia coli* W3110 | 0.001 | 0.005 | 0.003 | 0.003 | |
| W3110-aceA001 | 0.118 | 0.085 | 0.091 | 0.098 | P<0.01 |
| W3110-aceA002 | 0.138 | 0.095 | 0.111 | 0.115 | P<0.01 |
| CGMCC25402 | 75.33 | 74.91 | 75.15 | 75.130 | |
| YPR-aceA001 | 76.96 | 77.41 | 77.03 | 77.133 | P<0.01 |
| YPR-aceA002 | 77.59 | 77.04 | 77.23 | 77.287 | P<0.01 |

The above fermentation results indicated that both for a high-yield L-arginine-producing strain CGMCC25402 and wild-type *Escherichia coli* W3110, glutamine at position 371 in the amino acid sequence of the aceA gene was replaced by a termination codon or the aceA gene was completely knocked out, both of which were conducive to the increase in the yield of L-arginine.

### IV. L-tryptophan fermentation experiment

The *Escherichia coli* W3110 strain, L-tryptophan-producing bacteria CGMCC25403 and aceA engineered strains YPTrp-aceA001, W3110-aceA001, YPTrp-aceA002 and W3110-aceA002 were inoculated in a 5 L fermentation tank of BLBIO-5GC-4-H model (Shanghai Bailun Biotechnology Co., Ltd.), respectively. The fermentation experiment was carried out with an L-tryptophan fermentation medium according to culture conditions. This process was repeated three times for each strain. After the fermentation, the content of L-tryptophan was detected by high-performance liquid chromatography (HPLC), and the results were averaged from three replicates, as shown in Table 5.

L-tryptophan fermentation medium: water was used as a solvent; solute and its concentration were 7 g/L glucose, 1 g/L FM902 yeast powder, 1.2 g/L (NH4)₂SO₄, 1.2 g/L citric acid, 1.5 g/L MgSO₄·7H₂O, 5.5 g/L K₂HPO₄3H₂O, 0.2 mL/L defoamer, and ammonium introduced to adjust pH to 7.0.

L-tryptophan culture conditions:
corrected DO of 100%, temperature of 35°C, speed of 800 rpm, air volume of 5 L/min, and tank pressure of 0.00 Mpa;
inoculation amount: 10%;
initial conditions: temperature of 35°C, pH of 7.0, air volume of 1.0 L/min, and speed of 350 rpm; whole-process control: before the residual sugar was exhausted and the dissolved oxygen was ≤ 20%, the speed was successively increased to 400 rpm→450 rpm; after the residual sugar was exhausted, sugar was supplemented and the dissolved oxygen was controlled to 15%-30%; pH: 7.0 before 24 h and 6.7 after 24 h;
residual sugar control: 0.1% -0.5% before F12h; 0.1% -0.3% after F12h in combination with DO requirements;
material fed-batch: 25% of ammonia water, 55% of concentrated sugar, and 10% of Paodi; and fermentation cycle: about 34 h; and 15%-30% of dissolved oxygen was used in the control process as a standard for increasing and decreasing the air volume.

**Table 5: Yields of L-tryptophan**

| Strains | L-tryptophan | L-tryptophan | L-tryptophan | Mean | P-value |
|---|---|---|---|---|---|
| *Escherichia coli* W3110 | 0.007 | 0.011 | 0.004 | 0.007 | |
| W3110-aceA001 | 0.092 | 0.067 | 0.088 | 0.082 | P<0.01 |
| W3110-aceA002 | 0.082 | 0.085 | 0.094 | 0.087 | P<0.01 |
| CGMCC25403 | 37.71 | 38.38 | 38.06 | 38.050 | |
| YPTrp-aceA001 | 39.62 | 38.91 | 39.21 | 39.247 | P<0.01 |
| YPTrp-aceA002 | 39.01 | 39.68 | 38.95 | 39.213 | P<0.01 |

The above fermentation results indicated that both for a high-yield L-tryptophan-producing strain CGMCC25403 and wild-type *Escherichia coli* W3110, glutamine at position 371 in the amino acid sequence of the aceA gene was replaced by a termination codon or the aceA gene was completely knocked out, both of which were conducive to the increase in the yield of L-tryptophan.

### V. L-threonine fermentation experiment

The *Escherichia coli* W3110 strain, L-threonine-producing bacteria CGMCC25404 and aceA engineered strains YPTrp-aceA001, W3110-aceA001, YPTrp-aceA002 and W3110-aceA002 were inoculated in a 5 L fermentation tank of BLBIO-5GC-4-H model (Shanghai Bailun Biotechnology Co., Ltd.), respectively. The fermentation experiment was carried out with an L-threonine fermentation medium according to culture conditions. This process was repeated three times for each strain. After the fermentation, the content of L-threonine was detected by high-performance liquid chromatography (HPLC), and the results were averaged from three replicates, as shown in Table 6.

L-threonine fermentation medium: water was used as a solvent; solute and its concentration were 13 g/L glucose, 1 g/L (NH4)₂SO₄, 0.5 g/L of H₃PO₄, 0.8 g/L of KCl, 0.8 g/L of MgSO₄·7H₂O, 0.01 g/L of FeSO₄·7H₂O, 0.01 g/L of MnSO₄·H₂O, 1.5 g/L FM902 yeast powder, 5 g/L corn pulp, 17 g/L molasses, and ammonium introduced to adjust pH to 7.0.

L-threonine fermentation culture conditions:
corrected DO of 100%, temperature of 37°C, air volume of 5 L/min, speed of 800 rpm, tank pressure of 0 Mpa, and calibrated after 5min;
inoculation amount: 10%;
initial conditions: pH of 7.0, culture temperature of 37°C, tank pressure of 0 Mpa, air volume of 0.5 L/min, and speed of 400 rpm;
whole-process control: 1, the dissolved oxygen < 30%, and the speed was sequentially increased to 500 rpm-600 rpm→ air volume of 1 L/min→700 rpm→800 rpm; 2, the tank pressure was increased to 0.01 Mpa after 8 h of fermentation, and the tank pressure was increased to 0.02 Mpa-0.03 Mpa→ 0.04 Mpa-0.05 Mpa after 12 h of fermentation;
residual sugar control: 0.1% -0.5% before F12h; 0.1% -0.3% after F12h in combination with DO requirements;
material fed-batch: 25% of ammonia water, 55% of concentrated sugar, and 10% of Paodi; and
Fermentation cycle: about 30 h; and 20% -30% of dissolved oxygen was used in the control process as a standard for increasing and decreasing the air volume.

**Table 6: Yields of L-threonine**

| Strains | L-threonine | L-threonine | L-threonine | Mean | P-value |
|---|---|---|---|---|---|
| *Escherichia coli* W3110 | 0.009 | 0.007 | 0.017 | 0.011 | |
| W3110-aceA001 | 0.088 | 0.117 | 0.109 | 0.105 | P<0.01 |
| W3110-aceA002 | 0.093 | 0.071 | 0.125 | 0.096 | P<0.01 |
| CGMCC25404 | 98.59 | 98.13 | 97.92 | 98.213 | |
| YPThr-aceA001 | 99.79 | 98.95 | 99.62 | 99.453 | P<0.01 |
| YPThr-aceA002 | 99.17 | 99.41 | 98.96 | 99.180 | P<0.01 |

The above fermentation results indicated that both for a high-yield L-threonine-producing strain CGMCC25404 and wild-type *Escherichia coli* W3110, glutamine at position 371 in the amino acid sequence of the aceA gene was replaced by a termination codon or the aceA gene was completely knocked out, both of which were conducive to the increase in the yield of L-threonine. The present application is detailed above. For a person skilled in the art, the present application may be implemented within a wide range under the same parameters, concentrations and conditions without departing from the purpose and scope of the present application and without unnecessary experiments. Although special examples are given in the present application, it should be understood that further improvements may be made to the present application. In summary, according to the principles of the present application, the present application is intended to include any change, use or improvement of the present application, including changes that deviate from the scope disclosed in the present application and are made by conventional technologies known in the art. According to the scope of the claims attached below, some basic features can be applied.

### Industrial Applicability

The present application provides a use of an *Escherichia coli* isocitrate lyase gene aceA in the increase of the yield of L-amino acid in a microorganism. Experiments showed that the aceA gene and a variant thereof such as an aceA^{Q371*} gene participated in biosynthesis of L-amino acid, and knocking out or weakening the aceA gene, i.e., inhibiting or reducing the expression of the aceA gene, facilitated accumulation of L-amino acid. The aceA gene and the variant thereof (e.g., the aceA^{Q371*} gene) may be used to construct a genetically engineered strain for producing L-amino acid so as to greatly promote the increase of the yield of L-amino acid, and reduce the cost, which was of great significance to accelerate the process of L-amino acid industrialization.

Some of the sequences as used herein were as follows:
SEQ ID No. 1: ORF(CDS) nucleotide sequence (1305 bp) of wild-type aceA gene 
1        ATGAAAACCC GTACACAACA AATTGAAGAA TTACAGAAAG AGTGGACTCA ACCGCGTTGG
61       GAAGGCATTA CTCGCCCATA CAGTGCGGAA GATGTGGTGA AATTACGCGG TTCAGTCAAT
121      CCTGAATGCA CGCTGGCGCA ACTGGGCGCA GCGAAAATGT GGCGTCTGCT GCACGGTGAG
181      TCGAAAAAAG GCTACATCAA CAGCCTCGGC GCACTGACTG GCGGTCAGGC GCTGCAACAG
241      GCGAAAGCGG GTATTGAAGC AGTCTATCTG TCGGGATGGC AGGTAGCGGC GGACGCTAAC
301      CTGGCGGCCA GCATGTATCC GGATCAGTCG CTCTATCCGG CAAACTCGGT GCCAGCTGTG
361      GTGGAGCGGA TCAACAACAC CTTCCGTCGT GCCGATCAGA TCCAATGGTC CGCGGGCATT
421      GAGCCGGGCG ATCCGCGCTA TGTCGATTAC TTCCTGCCGA TCGTTGCCGA TGCGGAAGCC
481      GGTTTTGGCG GTGTCCTGAA TGCCTTTGAA CTGATGAAAG CGATGATTGA AGCCGGTGCA
541      GCGGCAGTTC ACTTCGAAGA TCAGCTGGCG TCAGTGAAGA AATGCGGTCA CATGGGCGGC
601      AAAGTTTTAG TGCCAACTCA GGAAGCTATT CAGAAACTGG TCGCGGCGCG TCTGGCAGCT
661      GACGTGACGG GCGTTCCAAC CCTGCTGGTT GCCCGTACCG ATGCTGATGC GGCGGATCTG
721      ATCACCTCCG ATTGCGACCC GTATGACAGC GAATTTATTA CCGGCGAGCG TACCAGTGAA
781      GGCTTCTTCC GTACTCATGC GGGCATTGAG CAAGCGATCA GCCGTGGCCT GGCGTATGCG
841      CCATATGCTG ACCTGGTCTG GTGTGAAACC TCCACGCCGG ATCTGGAACT GGCGCGTCGC
901      TTTGCACAAG CTATCCACGC GAAATATCCG GGCAAACTGC TGGCTTATAA CTGCTCGCCG
961      TCGTTCAACT GGCAGAAAAA CCTCGACGAC AAAACTATTG CCAGCTTCCA GCAGCAGCTG
1021     TCGGATATGG GCTACAAGTT CCAGTTCATC ACCCTGGCAG GTATCCACAG CATGTGGTTC
1081     AACATGTTTG ACCTGGCAAA CGCCTATGCC CAGGGCGAGG GTATGAAGCA CTACGTTGAG
1141     AAAGTGCAGC AGCCGGAATT TGCCGCCGCG AAAGATGGCT ATACCTTCGT ATCTCACCAG
1201     CAGGAAGTGG GTACAGGTTA CTTCGATAAA GTGACGACTA TTATTCAGGG CGGCACGTCT
1261     TCAGTCACCG CGCTGACCGG CTCCACTGAA GAATCGCAGT TCTAA
SEQ ID No. 2: amino acid sequence (434aa) of wild-type protein aceA 
MKTRTQQIEE LQKEWTQPRW EGITRPYSAE DVVKLRGSVN PECTLAQLGA AKMWRLLHGE61
SKKGYINSLG ALTGGQALQQ AKAGIEAVYL SGWQVAADAN LAASMYPDQS LYPANSVPAV121
VERINNTFRR ADQIQWSAGI EPGDPRYVDY FLPIVADAEA GFGGVLNAFE LMKAMIEAGA181
AAVHFEDQLA SVKKCGHMGG KVLVPTQEAI QKLVAARLAA DVTGVPTLLV ARTDADAADL241
ITSDCDPYDS EFITGERTSE GFFRTHAGIE QAISRGLAYA PYADLVWCET STPDLELARR301
FAQAIHAKYP GKLLAYNCSP SFNWQKNLDD KTIASFQQQL SDMGYKFQFI TLAGIHSMWF361
NMFDLANAYA QGEGMKHYVE KVQQPEFAAA KDGYTFVSHQ QEVGTGYFDK VTTIIQGGTS421
SVTALTGSTE ESQF*
SEQ ID No. 5: ORF(CDS) nucleotide sequence (1305 bp) of mutant aceA^{Q371*} gene 
1 ATGAAAACCC GTACACAACA AATTGAAGAA TTACAGAAAG AGTGGACTCA ACCGCGTTGG
61 GAAGGCATTA CTCGCCCATA CAGTGCGGAA GATGTGGTGA AATTACGCGG TTCAGTCAAT
121 CCTGAATGCA CGCTGGCGCA ACTGGGCGCA GCGAAAATGT GGCGTCTGCT GCACGGTGAG
181 TCGAAAAAAG GCTACATCAA CAGCCTCGGC GCACTGACTG GCGGTCAGGC GCTGCAACAG
241 GCGAAAGCGG GTATTGAAGC AGTCTATCTG TCGGGATGGC AGGTAGCGGC GGACGCTAAC
301 CTGGCGGCCA GCATGTATCC GGATCAGTCG CTCTATCCGG CAAACTCGGT GCCAGCTGTG
361 GTGGAGCGGA TCAACAACAC CTTCCGTCGT GCCGATCAGA TCCAATGGTC CGCGGGCATT
421 GAGCCGGGCG ATCCGCGCTA TGTCGATTAC TTCCTGCCGA TCGTTGCCGA TGCGGAAGCC
481 GGTTTTGGCG GTGTCCTGAA TGCCTTTGAA CTGATGAAAG CGATGATTGA AGCCGGTGCA
541 GCGGCAGTTC ACTTCGAAGA TCAGCTGGCG TCAGTGAAGA AATGCGGTCA CATGGGCGGC
601 AAAGTTTTAG TGCCAACTCA GGAAGCTATT CAGAAACTGG TCGCGGCGCG TCTGGCAGCT
661 GACGTGACGG GCGTTCCAAC CCTGCTGGTT GCCCGTACCG ATGCTGATGC GGCGGATCTG
721 ATCACCTCCG ATTGCGACCC GTATGACAGC GAATTTATTA CCGGCGAGCG TACCAGTGAA
781 GGCTTCTTCC GTACTCATGC GGGCATTGAG CAAGCGATCA GCCGTGGCCT GGCGTATGCG
841 CCATATGCTG ACCTGGTCTG GTGTGAAACC TCCACGCCGG ATCTGGAACT GGCGCGTCGC
901 TTTGCACAAG CTATCCACGC GAAATATCCG GGCAAACTGC TGGCTTATAA CTGCTCGCCG
961 TCGTTCAACT GGCAGAAAAA CCTCGACGAC AAAACTATTG CCAGCTTCCA GCAGCAGCTG
1021 TCGGATATGG GCTACAAGTT CCAGTTCATC ACCCTGGCAG GTATCCACAG CATGTGGTTC
1081 AACATGTTTG ACCTGGCAAA CGCCTATGCC TAGGGCGAGG GTATGAAGCA CTACGTTGAG
1141 AAAGTGCAGC AGCCGGAATT TGCCGCCGCG AAAGATGGCT ATACCTTCGT ATCTCACCAG
1201 CAGGAAGTGG GTACAGGTTA CTTCGATAAA GTGACGACTA TTATTCAGGG CGGCACGTCT
1261 TCAGTCACCG CGCTGACCGG CTCCACTGAA GAATCGCAGT TCTAA
SEQ ID No. 6: amino acid sequence (370aa) of mutant protein aceA^{Q371*}

MKTRTQQIEE LQKEWTQPRW EGITRPYSAE DVVKLRGSVN PECTLAQLGA AKMWRLLHGE61

SKKGYINSLG ALTGGQALQQ AKAGIEAVYL SGWQVAADAN LAASMYPDQS LYPANSVPAV121

VERINNTFRR ADQIQWSAGI EPGDPRYVDY FLPIVADAEA GFGGVLNAFE LMKAMIEAGA181

AAVHFEDQLA SVKKCGHMGG KVLVPTQEAI QKLVAARLAA DVTGVPTLLV ARTDADAADL241

ITSDCDPYDS EFITGERTSE GFFRTHAGIE QAISRGLAYA PYADLVWCET STPDLELARR301

FAQAIHAKYP GKLLAYNCSP SFNWQKNLDD KTIASFQQQL S[?]MGYKFQFI TLAGIHSMWF361

NMFDLANAYA

## Claims

1. A use of a protein or a substance that regulates the activity and/or content of the protein, wherein the use comprises any of the followings:
A1) a use in the construction of genetically engineered bacteria that produces L-amino acid;
A2) a use in the preparation of L-amino acid; and
A3) a use in the regulation of the yield of L-amino acid in a microorganism;
the protein comprises any of the followings:
B1) a protein comprising an amino acid sequence as shown in SEQ ID No. 2;
B2) a protein which is obtained by substitution and/or deletion and/or addition of an amino acid residue in the amino acid sequence as shown in SEQ ID No, 2, has the identity of 90% or more with the protein shown in B1) and has the same function as the protein shown in B1); and
B3) a fusion protein which is obtained by linking a tag to an N-terminal or/and a C-terminal of B1) or B2) and has the same function as that of B1) or B2).

2. The use of the nucleic acid molecule that codes the protein according to claim 1, wherein the use comprises any of the followings:
D1) a use in the construction of genetically engineered bacteria that produces L-amino acid;
D2) a use in the preparation of L-amino acid; and
D3) a use in the regulation of the yield of L-amino acid in a microorganism.

3. The use according to claim 2, wherein the nucleic acid molecule comprises any of the followings:
F1) a DNA molecule having a coding sequence as shown in SEQ ID No. 1; and
F2) a DNA molecule having a nucleotide sequence as shown in SEQ ID No. 1.

4. A use of the nucleic acid molecule in the inhibition or reduction of the expression of a coding gene of the protein according to claim 1, wherein the use comprises any of the followings:
D1) a use in the construction of genetically engineered bacteria that produces L-amino acid;
D2) a use in the preparation of L-amino acid; and
D3) a use in the regulation of the yield of L-amino acid in a microorganism.

5. The use according to claim 4, wherein the nucleic acid molecule comprises any of the followings:
H1) sgRNA1, having a target sequence as shown in SEQ ID No. 11; and
H2) sgRNA2, having a target sequence as shown in SEQ ID No. 12.

6. A use of an expression cassette containing the nucleic acid molecule according to claim 2 and/or claim 4, wherein the use comprises any of the followings:
D1) a use in the construction of genetically engineered bacteria that produces L-amino acid;
D2) a use in the preparation of L-amino acid; and
D3) a use in the regulation of the yield of L-amino acid in a microorganism.

7. A use of a recombinant vector containing any of the nucleic acid molecule according to claim 2 and/or claim 4, wherein the use comprises any of the followings:
D1) a use in the construction of genetically engineered bacteria that produces L-amino acid;
D2) a use in the preparation of L-amino acid; and
D3) a use in the regulation of the yield of L-amino acid in a microorganism.

8. A use of a recombinant microorganism containing the nucleic acid molecule according to claim 2 and/or claim 4, wherein the use comprises any of the followings:
D1) a use in the construction of genetically engineered bacteria that produces L-amino acid;
D2) a use in the preparation of L-amino acid; and
D3) a use in the regulation of the yield of L-amino acid in a microorganism.

9. A use of a recombinant host cell containing the nucleic acid molecule according to claim 2 and/or claim 4, wherein the use comprises any of the followings:
D1) a use in the construction of genetically engineered bacteria that produces L-amino acid;
D2) a use in the preparation of L-amino acid; and
D3) a use in the regulation of the yield of L-amino acid in a microorganism.

10. A method for increasing the yield of L-amino acid in a microorganism, the method comprising: reducing the content and/or activity of the protein according to claim 1 in a target microorganism to obtain a microorganism with a higher yield of L-amino acid than the target microorganism.

11. The method according to claim 11, wherein the reducing the content and/or activity of the protein according to claim 1 in the target microorganism is implemented by reducing the expression quantity and/or activity of the coding gene of the protein in the target microorganism.

12. The method according to claim 11, wherein the reducing the expression quantity and/or activity of the coding gene of the protein in the target microorganism refers to reducing or inactivating the activity of the coding gene of the protein according to claim 1 in a genome of the target microorganism by using a gene mutation, gene knockout, gene editing or gene attenuation technology.

13. The method according to claim 12, wherein the reducing or inactivating the activity of the coding gene of the protein according to claim 1 in the genome of the target microorganism by using the gene editing technology is performed by using a CRISPR/Cas9 system; the CRISPR/Cas9 system comprises a vector that expresses sgRNA targeting the coding gene of the protein; and the sgRNA comprises any of the followings:
G1) sgRNA1 for gene mutation, having a target sequence as shown in SEQ ID No. 11; and
G2) sgRNA2 for gene knockout, having a target sequence as shown in SEQ ID No. 12.

14. The method according to claim 13, wherein the gene mutation in G1) is the mutation of a DNA molecule having a nucleotide sequence of SEQ ID No. 1 in the target microorganism into a DNA molecule as shown in SEQ ID No. 5.

15. A recombinant microorganism, in which the protein according to claim 1 is weakly expressed or not expressed in the recombinant microorganism.

16. The recombinant microorganism according to claim 15, wherein the weak expression or no expression is implemented by reducing the expression quantity and/or activity of the coding gene of the protein in the target microorganism.

17. The recombinant microorganism according to claim 16, wherein the reducing the expression quantity and/or activity of the coding gene of the protein in the target microorganism refers to reducing or inactivating the activity of the coding gene of the protein according to claim 1 in a genome of the target microorganism by using a gene mutation, gene knockout, gene editing or gene attenuation technology.

18. The recombinant microorganism according to claim 17, wherein the gene editing is performed by using a CRISPR/Cas9 system; the CRISPR/Cas9 system comprises a vector that expresses sgRNA targeting the coding gene of the protein; and the sgRNA comprises any of the followings:
G1) sgRNA1 for gene mutation, having a target sequence as shown in SEQ ID No. 11; and
G2) sgRNA2 for gene knockout, having a target sequence as shown in SEQ ID No. 12.

19. The recombinant microorganism according to claim 18, wherein the gene mutation in G1) is the mutation of a DNA molecule having a nucleotide sequence of SEQ ID No. 1 in the target microorganism into a DNA molecule as shown in SEQ ID No. 5.

20. A recombinant microorganism, wherein the recombinant microorganism contains a nucleic acid molecule that inhibits or reduces the expression of a coding gene of the protein according to claim 1.

21. The recombinant microorganism according to claim 20, wherein the nucleic acid molecule comprises any of the followings:
H1) sgRNA1, having a target sequence as shown in SEQ ID No. 11; and
H2) sgRNA2, having a target sequence as shown in SEQ ID No. 12.

22. A protein, comprising any of the followings:
M1) a protein comprising an amino acid sequence as shown in SEQ ID No. 6;
M2) a protein which is obtained by substitution and/or deletion and/or addition of an amino acid residue in the amino acid sequence as shown in SEQ ID No, 6, has the identity of 80% or more with the protein shown in M1) and has the same function as the protein shown in M1); and
M3) a fusion protein which is obtained by linking a tag to an N-terminal and/or a C-terminal of M1) or M2) and has the same function as that of M1) or M2).

23. A nucleic acid molecule that codes the protein according to claim 22.

24. An expression cassette containing a nucleic acid molecule that codes the protein according to claim 22.

25. A recombinant vector containing a nucleic acid molecule that codes the protein according to claim 22.

26. A recombinant microorganism, containing a nucleic acid molecule that codes the protein according to claim 22.

27. A recombinant host cell, containing the nucleic acid molecule that codes the protein according to claim 22;

28. The nucleic acid molecule according to any one of claims 23 to 27, wherein the nucleic acid molecule comprises the DNA molecule as shown in SEQ ID No. 5.

29. A use of the protein according to claim 22 or the nucleic acid molecule according to any one of claims 23 to 28 in the followings:
N1) a use in the construction of genetically engineered bacteria that produces L-amino acid;
N2) a use in the preparation of L-amino acid; and
N3) a use in the regulation of the yield of L-amino acid in a microorganism.

30. The use according to any of claims 1 to 9 and 29 and the method according to any of claims 10 to14, wherein the L-amino acid comprises L-alanine, L-valine, L-arginine, L-tryptophan, and/or L-threonine.
